# EUROPEAN PATENT APPLICATION

(11) **EP 2 366 804 A1**
(43) Date of publication of application: **21.09.2011**
(21) Application number: 10358001.5
(22) Date of filing: 02.03.2010
(51) Int. Cl.: C12Q 1/70

(54) **Identification of the microVar strain of Ostreid herpesvirus 1 (OsHV-1)**

(71) Applicant: IFREMER (INSTITUT FRANCAIS DE RECHERCHE POUR L'EXPLOITATION DE LA MER), 92138 Issy les Moulineaux Cedex (FR)
(72) Inventor: Pepin, Jean-Francois, 17620 Saint Agnant (FR); Renault, Tristan, 17300 Rochefort sur Mer (FR); Segarra, Amélie, 77470 Fublaines (FR); Morga, Benjamin, 47370 Tournon d'Agenais (FR)
(74) Representative: Barbot, Willy

(57) **Abstract**

The present invention concerns a method for determining if a particular Ostreid herpesvirus 1 (OsHV-1) corresponds to the OsHV-1 µVar strain, which method comprises the steps of (i) determining the sequence of the C region (SEQ ID n°1) and/or of the IA region (SEQ ID n°2) of the genomic DNA of said OsHV-1 virus, and (ii) determining if said OsHV-1 virus corresponds to the OsHV-1 µVar strain; and a pair of primers and a kit useful for such a method.

SEQUENCE LISTING

<110> IFREMER
PEPIN, Jean-François
SEGARRA, Amélie
MORGA, Benjamin
RENAULT, Tristan

<120> Identification of the Var strain of Ostreid herpesvirus 1
(OsHV-1)

<130> IFR-B-0003EP

<160> 9

<170> PatentIn version 3.5

<210> 1
<211> 672
<212> DNA
<213> Crassostrea gigas

<220>
<221> variation
<222> (151)..(151)
<223> n= A or G

<220>
<221> variation
<222> (223)..(223)
<223> n= C or AA

<220>
<221> variation
<222> (249)..(249)
<223> n= GC or nothing

<220>
<221> variation
<222> (253)..(253)
<223> n= G or C

<220>
<221> variation
<222> (257)..(257)
<223> n= A or G

<220>
<221> variation
<222> (306)..(306)
<223> n= AC or nothing

<220>
<221> variation
<222> (360)..(360)
<223> n= G or ACTACTACTACTGA

<220>
<221> variation
<222> (482)..(482)
<223> n= A or G

<220>
<221> variation
<222> (488)..(488)
<223> n= A or G

<400>  1
caccaatgtg gtaaagacgg aacaatcttt ttctaggata tggagctgcg gcgctatgga      60

tttaacgagt gccaccaaaa gttgggataa tgattttaga atagatgtga tgtgcggcaa     120

gatgaatggc aagatacaca atgagctatt ncccgaccac aaacctaacg ttgtattcga     180

ttacggatta agaaaatggg ttccacaatc taaaattaaa aanccacatg ggggccaagg     240

aatttaaanc ccngggnaaa aagtataaat aggcgcgatt tgtcagttta gaatcatacc     300

cacacntcaa tctcgagtat accacaactg ctaaattaac agcatctact actactactn     360

aaaaatgcag cctttcacag aattttgcac cttgaccaaa gccatcacat cagccagcaa     420

cgactttttc atcaaccaga cgaggttaac atgcgacatt tgtaaagagc tcgtctcttt     480

cnattgcnaa gataaagtcg tggcatcatt ggctgcagtc agatctgaca tacccataga     540

agtcacggaa cgcaaagacc tgaacctcct cgacctgatc cagttcttcg aaaagaagat     600

agagtttacc actctcattg acgaattgtt cactgcccac aaagaccatt gtcagaaaat     660

ggtaagccgt gc                                                         672

<210>  2
<211>  608
<212>  DNA
<213>  Crassostrea gigas

<220>
<221>  variation
<222>  (110)..(110)
<223>  n= A or no nucleic acid

<220>
<221>  variation
<222>  (526)..(526)
<223>  n= C or T

<400>  2
aatccccatg tttcttgctg tagaataatt tgctatctga tttggtttat atttttgta      60

aagcttttat atatcttcaa atccggaagt gttttaacaa caagattacn aaaaaatatc     120

aacggcaatg tctaatttgt tcattccccg atctaccaaa cgtgcagtct acgacggccc     180

tttgccaatg gtaggctctt ccctgccgcc aatagaaata aacagcaaag gtgataaatc     240

ggtagtttat ctcaggggtg atgatcaacc aattgatgtt aacagggaac atagaatggt     300

aaaagttacg tataatgaat acgatgagca agaaacgatc aaggttattt tcctcgacaa     360

gaaagcaaca ataaaagatc tacataacct aatgagtgtt ggtagggatc ttacaacggg     420

tgtctgcaat atagaagtac aaccggaata tggattcaca ctgaggatac cagacccaga     480

caagttgaaa tataaaagtg atatagatgc agtctataga ctcttngctt caaaatacga     540

caatagcgat ctattcgaaa gggcatcaga gtcattagcg tttcaaataa ctttggatat     600

gaaccgcg                                                              608

<210>  3

<211>  14
<212>  DNA
<213>  Crassostrea gigas

<400>  3
actactacta ctga                                                    14


<210>  4
<211>  672
<212>  DNA
<213>  Crassostrea gigas

<400>  4
caccaatgtg gtaaagacgg aacaatcttt ttctaggata tggagctgcg gcgctatgga      60

tttaacgagt gccaccaaaa gttgggataa tgattttaga atagatgtga tgtgcggcaa     120

gatgaatggc aagatacaca atgagctatt acccgaccac aaacctaacg ttgtattcga     180

ttacggatta agaaaatggg ttccacaatc taaaattaaa aacccacatg ggggccaagg     240

aatttaaagc cccggggaaa aaagtataaa taggcgcgat ttgtcagttt agaatcatac     300

ccacactcaa tctcgagtat accacaactg ctaaattaac agcatctact actactactg     360

aaaaatgcag cctttcacag aattttgcac cttgaccaaa gccatcacat cagccagcaa     420

cgactttttc atcaaccaga cgaggttaac atgcgacatt tgtaaagagc tcgtctcttt     480

caattgcaaa gataaagtcg tggcatcatt ggctgcagtc agatctgaca tacccataga     540

agtcacggaa cgcaaagacc tgaacctcct cgacctgatc cagttcttcg aaaagaagat     600

agagtttacc actctcattg acgaattgtt cactgcccac aaagaccatt gtcagaaaat     660

ggtaagccgt gc                                                      672


<210>  5
<211>  607
<212>  DNA
<213>  Crassostrea gigas

<400>  5
aatccccatg tttcttgctg tagaataatt tgctatctga tttggtttat atttttgta      60

aagcttttat atatcttcaa atccggaagt gttttaacaa caagattaca aaaaatatca     120

acggcaatgt ctaatttgtt cattccccga tctaccaaac gtgcagtcta cgacggccct     180

ttgccaatgg taggctcttc cctgccgcca atagaaataa acagcaaagg tgataaatcg     240

gtagtttatc tcaggggtga tgatcaacca attgatgtta acagggaaca tagaatggta     300

aaagttacgt ataatgaata cgatgagcaa gaaacgatca aggttatttt cctcgacaag     360

aaagcaacaa taaaagatct acataaccta atgagtgttg gtagggatct tacaacgggt     420

gtctgcaata tagaagtaca accggaatat ggattcacac tgaggatacc agacccagac     480

aagttgaaat ataaaagtga tatagatgca gtctatagac tctttgcttc aaaatacgac     540

aatagcgatc tattcgaaag ggcatcagag tcattagcgt ttcaaataac tttggatatg     600

aaccgcg                                                            607

<210>  6
<211>  349
<212>  PRT
<213>  Crassostrea gigas

<220>
<221>  VARIANT
<222>  (40)..(40)
<223>  Xaa= D or N

<220>
<221>  VARIANT
<222>  (42)..(42)
<223>  Xaa= E or K

<400>  6

Met Gln Pro Phe Thr Glu Phe Cys Thr Leu Thr Lys Ala Ile Thr Ser
1               5                   10                  15

Ala Ser Asn Asp Phe Phe Ile Asn Gln Thr Arg Leu Thr Cys Asp Ile
            20                  25                  30

Cys Lys Glu Leu Val Ser Phe Xaa Cys Xaa Asp Lys Val Val Ala Ser
        35                  40                  45

Leu Ala Ala Val Arg Ser Asp Ile Pro Ile Glu Val Thr Glu Arg Lys
    50                  55                  60

Asp Leu Asn Leu Leu Asp Leu Ile Gln Phe Phe Glu Lys Lys Ile Glu
65                  70                  75                  80

Phe Thr Thr Leu Ile Asp Glu Leu Phe Thr Ala His Lys Asp His Cys
                85                  90                  95

Gln Lys Asn Gly Lys Pro Cys Thr Met Leu Ser Ile Ile Ala Gly Arg
            100                 105                 110

Tyr Thr Asp Glu Leu Tyr Arg Glu Glu Ser Ile Glu Glu Asn Arg Ser
        115                 120                 125

Trp Val Leu Lys Ala Tyr Lys Leu Asp Leu Ile Asn Gln Gln Phe Phe
    130                 135                 140

Arg Asn Asn Arg Leu Tyr Asp Ile Leu Lys Glu Ala Gly Val Glu Asn
145                 150                 155                 160

Leu Glu Arg Ile Met Leu Glu Asp Glu Ile Glu Ser Val Cys Lys Thr
                165                 170                 175

Lys Ala Lys Ser Pro Glu Glu Val Ile Thr Lys Met Ile Pro Glu Gln
            180                 185                 190

Leu Thr Lys Leu Leu Glu Leu Met Ser Cys Pro Gln Lys Gln Val Thr
        195                 200                 205

Val Tyr Ile Arg Gly Ser Gly Tyr Thr Pro Val Asn His Ala Lys Leu
    210                 215                 220

Asn Ala Cys Leu Lys Asp Arg Asp Ile Gln Leu Tyr Leu Gly Met Pro
225                 230                 235                 240

Met Ser Tyr Phe Arg Tyr Ser Ser Trp Glu Ser Tyr Tyr Glu Gly Asn
                245                 250                 255

Gly Met Cys Leu Lys Ser Glu His Glu Leu Lys His Asn Val Ala Lys
            260                 265                 270

Val Tyr Ala Ala Val Lys Lys Val Ala Pro Lys Val Lys Leu Leu Ala
        275                 280                 285

Trp Thr Leu Glu Ala Asn Asp Glu Met Arg Thr Cys Ser Cys Met Val
    290                 295                 300

Lys Gln His Asp Met Ala Ile Ala Ala Ile Arg Gly Glu Leu Tyr Val
305                 310                 315                 320

Pro Pro Glu Leu Arg Pro Val Pro Val Arg Leu Gly Lys Arg Arg Ala
                325                 330                 335

Ser Ser Gly Asp Val Ser Asp Ile Pro Met Lys Arg Asn
            340                 345

<210>  7
<211>  20
<212>  DNA
<213>  artificial sequence

<220>
<223>  CR primer

<400>  7
gtgatggctt tggtcaaggt                                                   20

<210>  8
<211>  20
<212>  DNA
<213>  artificial sequence

<220>
<223>  CF primer

<400>  8
ccccggggaa aaagtataaa                                                   20

<210>  9
<211>  158
<212>  DNA
<213>  Crassostrea gigas

<400>  9

cccggggaaa aaagtataaa taggcgcgat ttgtcagttt agaatcatac ccacactcaa 60
tctcgagtat accacaactg ctaaattaac agcatctact actactactg aaaaatgcag 120
cctttcacag aattttgcac cttgaccaaa gccatcac 158

## Description

### Field of the invention

The present invention relates to mollusc infection by Herpes-like viral and is directed to oligonucleotides, methods and kits which are especially useful as an aid in the diagnosis of an infection with the µVar strain of Ostreid herpesvirus 1 (OsHV-1).

### Background of the invention

Herpes-like viral infections have been reported in different bivalve mollusc species throughout the world. More specifically, Ostreid herpesvirus 1 (OsHV-1) is the only member of the Herpesviridae that has an invertebrate host and is associated with sporadic mortality in the Pacific oyster (*Crassostrea gigas*) and other bivalve species.

Following the characterisation and genome sequencing of ostreid herpesvirus 1 (OsHV-1), specific diagnostic tools have been developed based on conventional PCR techniques, in situ hybridisation and quantitative PCR.

In France, the year 2008 displayed high mortality rate ranging from 80% to 100% affecting only cupped oyster, *Crassostrea gigas* and mainly juvenile oysters from May to September.

According to devastating of losses, it appeared important to investigate if this high mortality rate could be associated to emerging or new pathogens.

Because of preliminary tests for herpesvirus detection showed around 75% positive batches; the question was whether it could be related to the emergence of a new genotype of Ostreid Herpesvirus type 1 (OsHV-1).

### Summary of the invention

The present invention relates to a method for determining if a particular Ostreid herpesvirus 1 (OsHV-1) corresponds to the OsHV-1 µVar strain, which method comprises the step of (i) determining the sequence of the C region (SEQ ID n°1) and/or of the IA region (SEQ ID n°2) of the genomic DNA of said OsHV-1 virus, and (ii) determining if said OsHV-1 virus corresponds to the OsHV-1 µVar strain.

The present invention further relates to a pair of oligobucleotides useful for determining if a particular Ostreid herpesvirus 1 (OsHV-1) corresponds to the OsHV-1 µVar strain, wherein said pair of oligobucleotides have the sequences GTG ATG GCT TTG GTC AAG GT (SEQ ID n°7) and CCC CGG GGA AAA AGT ATA AA (SEQ ID n°8).

Finally, the present invention relates to a kit for determining if a particular Ostreid herpesvirus 1 (OsHV-1) corresponds to the OsHV-1 µVar strain.

### Brief description of the drawings

The figure 1 shows the identified polymorphisms in C2/C6 fragment associated with increased mortality.
The figure 2 shows the ORF4 amino acids changes associated with OsHV-1 µVar sequence.
The figure 3 shows identified polymorphisms in IA1-IA2 fragments also associated with OsHV-1 µvar sequence.
The figure 4 shows an electrophoresis gel of PCR products obtained with CF/CR primers realized on 2 OsHV-1 isolates and 2 *OsHV-1 µVar* isolates. Only two genotype µVar (1a and 12) and two genotype reference OsHV-1 (3 and 4) are presented. C: positive control. M: DNA ladder.

### Detailed description

The inventors have now identified a new OsHV-1 genotype or strain in 40% of studied spat batches from 2008 called OsHV-1 µVar, which could be considered as an emerging viral genotype involved in oyster mortalities.

Specific polymorphisms of this genotype have been identified by the inventors, which polymorphisms are located in two targeted regions C and IA, with reference to OsHV-1 DNA.

These polymorphisms correspond as an example to a deletion in a microsatellite zone and to substitutions in coding zones of the ORF4 protein.

Finally, the inventors have identified a PCR primers pair enabling a simple and fast diagnosis of oyster OsHV-1 µVar infection.

Consequently, the present invention relates to a method for determining if a particular Ostreid herpesvirus 1 (OsHV-1) corresponds to the OsHV-1 µVar strain, which method comprises the step of:
i) determining the sequence of the C region (SEQ ID n°1) and/or of the IA region (SEQ ID n°2) of the genomic DNA of said OsHV-1 virus, and
ii) determining if said OsHV-1 virus corresponds to the OsHV-1 µVar strain.

Said particular OsHV-1 virus is isolated or not from a biological sample.

As used herein, said biological sample can correspond to plankton, mollusc or seawater.

The step of determining if said OsHV-1 virus corresponds to a OsHV-1 µVar strain in view of its sequence can be simply done by the skilled person in view of the polymorphisms identified by the inventors.

In fact, the inventors have established that the following nucleic acid polymorphisms are characteristic of the OsHV-1 µVar strain as compared to the reference DNA of OsHV-1:
● For SEQ ID n°1, at least an A instead of a G at position 151, a C instead of AA at position 223, an insertion of GC at position 249 instead of no nucleic acid, a G instead of C at position 253, an A instead of G at position 257, no nucleic acid instead of AC at position 306, G instead of ACTACTACTACTGA (SEQ ID n°3) at position 360, A instead of G at position 482, and/or a A instead of G at position 488.
● For SEQ ID n°2, no nucleic acid instead of an A at position 110 and/or a T instead of a C at position 526.

Thus, the sequences SEQ ID n°4 and SEQ ID n°5 are characteristic of the C and IA regions respectively of the µVar C genotype.

It should be further noticed that, as a result of some of the previously disclosed nucleic acid polymorphisms, the following amino acid polymorphisms are characteristic of the OsHV-1 µVar strain as compared to the reference DNA of OsHV-1:
● For ORF4 (SEQ ID n°6), at least a N instead of a D at position 40 and/or a K instead of a E at position 42.

Advantageously, said determining step (ii) is done by identifying at least one of the previously disclosed polymorphisms.

In a preferred embodiment, said method comprises the step of determining the sequence of the C region (SEQ ID n°1) of the genomic DNA of said OsHV-1 virus.

In another preferred embodiment, said determining step (ii) is done by identifying the polymorphism corresponding to G instead of ACTACTACTACT6A (SEQ ID n°3) at position 360 of the C region (SEQ ID n°1) of the genomic DNA of said OsHV-1 virus.

In a still preferred embodiment, the method of the invention is a method for diagnosis an infection by an OsHV-1 µVar strain in a mollusc, wherein said method further comprises a previous step of determining the presence or the absence of an OsHV-1 virus in a biological sample of said mollusc.

Thus, the biological sample is a mollusc, or a part thereof, and most preferably a soft tissue of said mollusc.

The step of determining the presence or the absence of an OsHV-1 virus in a biological sample of said mollusc can be done by well known methods such as those disclosed in PEPIN et all. (J VirolMethods, vol.149(2), p:269-76, 2008).

As used herein, a mollusc is a bivalve mollusc selected in the group comprising oysters, scallops and clams.

Preferably, said mollusc is an oyster and most preferably a cupped oyster such as *Crassostrea gigas*

The step (i) of determining the sequence of the C region (SEQ ID n°1) and/or of the IA region (SEQ ID n°2) of the genomic DNA of said OsHV-1 virus can be done directly by sequencing of these regions or indirectly by hybridization techniques such as DNA array or Southern blot, by enzymatic digestion techniques such as RFLP, by amplification techniques such as Polymerase Chain Reaction (PCR), Southern Blot, Northern Blot, or by enzymatic techniques such as PRA (PCR-restriction length polymorphism analysis).

Preferably, said determining step (i) is done by amplification techniques and most preferably by PCR.

Useful primers for said amplification techniques have a length of at least 12 nucleic acids, preferably of at least 15 nucleic acids and most preferably of at least 20 nucleic acids.

In a still preferred embodiment, the determining step (i) is done by PCR using the pair of primers CR (GTG ATG GCT TTG GTC AAG GT, SEQ ID n°7) and CF (CCC CGG GGA AAA AGT ATA AA, SEQ ID n°8).

Said pair of primers enables a simple and fast identification of a OsHV-1 virus corresponding to a µVar OsHV-1 strain by PCR, since samples comprising DNA of said strain result in a 158pb amplicon (SEQ ID n°9).

A second aspect of the invention concerns a pair of oligobucleotides useful for determining if a particular Ostreid herpesvirus 1 (OsHV-1) corresponds to the OsHV-1 µVar strain, wherein said pair of oligobucleotides have the sequences GTG ATG GCT TTG GTC AAG GT. (SEQ ID n°7) and (CCC CGG GGA AAA AGT ATA AA (SEQ ID n°8).

A third aspect of the invention concerns a kit for determining if a particular Ostreid herpesvirus 1 (OsHV-1) corresponds to the OsHV-1 µVar strain, wherein said kit comprises:
● means for determining the sequence of the C region (SEQ ID n°1) and/or of the IA region (SEQ ID n°2) of the genomic DNA of said OsHV-1 virus.

As an example, such means can include primers for amplifying the C region (SEQ ID n°1) and/or of the IA region (SEQ ID n°2) of the genomic DNA of said OsHV-1 virus, or antibodies recognizing the ORF4 protein specifically expressed by the OsHV-1 µVar strain comprising at least a N instead of a D at position 40 and a K instead of a E at position 42 of ORF4 (SEQ ID n°6).

Advantageously, said means corresponds to the previously described pair of oligonucleotides.

The kit of the invention may comprise additional reagents useful for the extraction of nucleic acids from a tissue sample or for analyzing the nucleic acid extracted from a tissue sample such as polymerase chain reaction reagents. The kit may also comprise specific controls based on purified plasmidic DNA from clones containing insert C2/C6 fragments as targeted DNA matrix originating from reference or µVar genotype Oshv-1 isolates.

The following experiments are offered to illustrate embodiments of the invention and should not be viewed as limiting the scope of the invention.

### Examples

### Identification of a new genotype of OsHV-1 associated with increased Oyster mortality

DNA was extracted from Oyster soft tissue as disclosed in PEPIN et *al.* (abovementioned, 2008).

The used DNA corresponds to 24 batches from field mortality events >70% in 2008, 4 batches previously used in study of challenge in 2008 and to 49 DNA samples from several years and different countries.

OsHV-1 DNA were identified in said samples and regions of said OsHV-1 DNAs were sequenced.

The analysis of said sequences as compared with OsHV-1 reference DNA (AY509253, GenBank) revealed several polymorphisms in ORF4 and ORF43 regions.

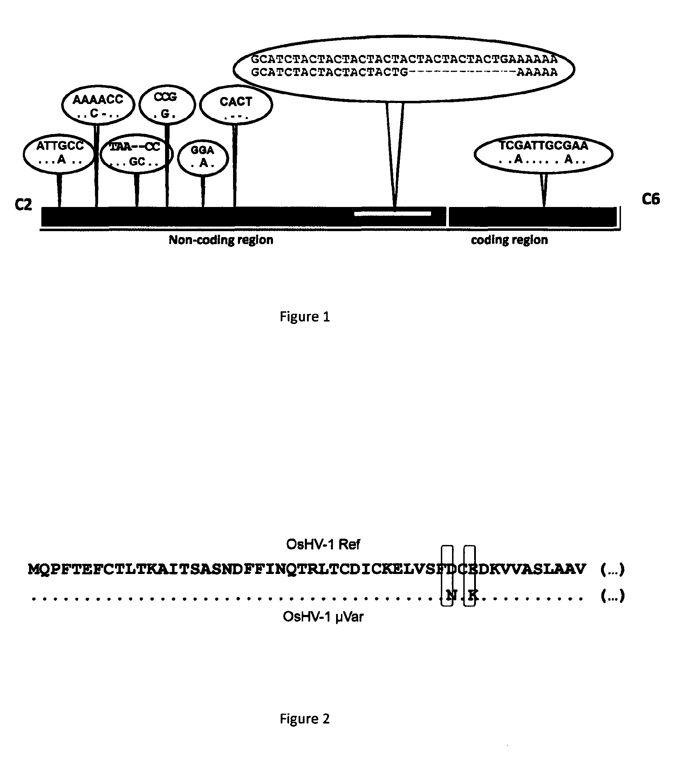

The figure 1 shows the identified polymorphisms in C2/C6 fragment associated with increased mortality.

These nucleic acids polymorphisms result in ORF4 amino acids changes as shown in Figure 2.

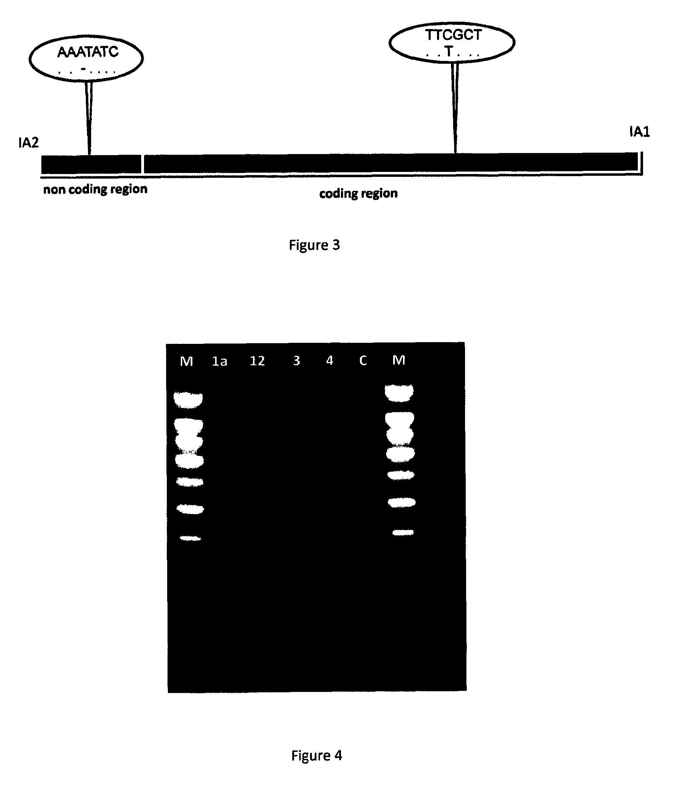

The figure 3 shows identified polymorphisms in IA1-IA2 fragments also associated with increased mortality.

These polymorphisms are specific of the new OsHV-1 genotype (strain) µVar and their detections enable to diagnose an infection with this specific genotype.

### Identification of a new genotype of OsHV-1 associated with increased Oyster mortality

DNA was extracted from Oyster larvae infected with OsHV-1 µVar genotype. DNA is then diluted to a concentration comprised between 10 end 500ng DNA/*µ*l

As a positive control, OsHV-1 reference genomic DNA (Accession Number : AY 509253) was used, which corresponds to the one purified from oyster larvae infected in 1995 by OsHV-1 virus. The concentration of this reference OsHV-1 genomic DNA was about 2 pg/µl corresponding to 50.10³ DNA copies/*µ*l.

So as to identify OsHV-1 µVar genotype, specific primers CR (GTG ATG GCT TTG GTC AAG GT, SEQ ID n°7) and CF (CCC CGG GGA AAA AGT ATA AA, SEQ ID n°8) have been designed.

The following melting on the basis of MIX RED GOLD STAR kit (EUROGENTEC) was prepared:

| Compound | Volume |
|---|---|
| Buffer 10x | 5µl |
| MgC1₂ (25mM) | 5µl |
| dNTP (2mM) | 5µl |
| Primer CR (10µM) | 1µl |
| Primer CF (10µM) | 1µl |
| Taq RED (5U/µl) | 0,5µl |
| DNA sample (10 to 500 ng/ *µ*l) | 1µl |
| H₂O | qs 50µl |

The following PCR program was used:

| Temperature (°*C*) | Time (min) | Number of cycles |
|---|---|---|
| 94 | 2 | 1 |
| 94 | 1 | 35 |
| 50 | 1 | |
| 72 | 1 | |
| 72 | 5 | 1 |

The obtained PCR products are loaded on a 2.5% Agarose electrophoresis gel.

The figure 4 shows an electrophoresis gel of PCR products obtained with CF/CR primers realized on 2 OsHV-1 isolates and 2 OsHV-1 µVar isolates. Only two genotype µVar (1a and 12) and two genotype reference OsHV-1 (3 and 4) are presented. C: positive control. M: DNA ladder.

The results show that the *µ*Var OsHV-1 strain samples show a 158 pb amplicon, whereas the reference OsHV-1 strain samples show a 173 pb amplicon enabling a simple and fast characterisation of OsHV-1 µVar strain *(See* fig 4).

## Claims

1. A method for determining if a particular Ostreid herpesvirus 1 (OsHV-1) corresponds to the OsHV-1 µVar strain, which method comprises the step of:
i) determining the sequence of the C region (SEQ ID n°1) and/or of the IA region (SEQ ID n°2) of the genomic DNA of said OsHV-1 virus, and
ii) determining if said OsHV-1 virus corresponds to the OsHV-1 µVar strain.

2. The method of claim 1, wherein said determining step (ii) is done by identifying at least one nucleic acid polymorphism characteristic of the OsHV-1 µVar strain selected in the group comprising:
● For SEQ ID n°1, at least an A instead of a G at position 151, a C instead of AA at position 223, an insertion of GC at position 249 instead of no nucleic acid, a G instead of C at position 253, an A instead of G at position 257, no nucleic acid instead of AC at position 306, G instead of ACTACTACTACTGA (SEQ ID n°3) at position 360, A instead of G at position 482, and/or a A instead of G at position 488.
● For SEQ ID n°2, no nucleic acid instead of an A at position 110 and/or a T instead of a C at position 526.

3. The method of any of claim 1 or 2, wherein said method comprises the step of determining the sequence of the C region (SEQ ID n°1) of the genomic DNA of said OsHV-1 virus.

4. The method of claim 3, wherein the determining step (ii) is done by identifying the polymorphism corresponding to G instead of ACTACTACTACTGA (SEQ ID n°3) at position 360 of the C region (SEQ ID n°1) of the genomic DNA of said OsHV-1 virus.

5. The method of any of claims 1 to 4, wherein said method is for diagnosing an infection by an OsHV-1 µVar strain in a mollusc, wherein said method further comprises a previous step of determining the presence or the absence of an OsHV-1 virus in a biological sample of said mollusc, preferably said biological sample is a soft tissue of said mollusc.

6. The method of claim 5, wherein said mollusc is an oyster and preferably *Crassostrea gigas*

7. The method of any of claims 1 to 6, wherein the step (i) of determining the sequence of the C region (SEQ ID n°1) and/or of the IA region (SEQ ID n"2) of the genomic DNA of said OsHV-1 virus is done by nucleic acid amplification techniques and preferably by PCR.

8. The method of claim 7, wherein the determining step (i) is done by PCR using the pair of primers CR (GTG ATG GCT TTG GTC AAG GT, SEQ ID n°7) and CF (CCC CGG GGA AAA AGT ATA AA, SEQ ID n°8).

9. A pair of oligonucleotides useful for determining if a particular Ostreid herpesvirus 1 (OsHV-1) corresponds to the OsHV-1 µVar strain, wherein said pair of oligonucleotides have the sequences GTG ATG GCT TTG GTC AAG GT (SEQ ID n°7) and (CCC CGG GGA AAA AGT ATA AA (SEQ ID n°8).

10. A kit for determining if a particular Ostreid herpesvirus 1 (OsHV-1) corresponds to the OsHV-1 µVar strain, wherein said kit comprises the pair of oligonucleotides as defined in claim 10.

## Amended claims

### Amended claims in accordance with Rule 137(2) EPC.

**1.** A method for determining if a particular Ostreid herpesvirus 1 (OsHV-1) corresponds to the OsHV-1 µVar strain, which method comprises the step of:
i) determining the sequence of the C region of the genomic DNA of said OsHV-1 virus, wherein said C region has the sequence SEQ ID n°1 and
ii) determining if said OsHV-1 virus corresponds to the OsHV-1 µVar strain by identifying the polymorphism corresponding to G instead of ACTACTACTACTGA (SEQ ID n°3) at position 360 of the C region of the genomic DNA of said OsHV-1 virus.

**2.** The method of claim 1, wherein said method is for diagnosing an infection by an OsHV-1 µVar strain in a mollusc, wherein said method further comprises a previous step of determining the presence or the absence of an OsHV-1 virus in a biological sample of said mollusc.

**3.** The method of claim 2, wherein said biological sample is a soft tissue of said mollusc.

**4.** The method of claim 2, wherein said mollusc is an oyster.

**5.** The method of claim 4, wherein said oyster is *Crossostrea gigas.*

**6.** The method of any of claims 1 to 5, wherein the step (i) of determining the sequence of the C region of the genomic DNA of said OsHV-1 virus is done directly by sequencing this region or indirectly by hybridization techniques, by enzymatic digestion techniques, by amplification techniques or by enzymatic techniques.

**7.** The method of claim 6, wherein the step (i) of determining the sequence of the C region of the genomic DNA of said OsHV-1 virus is done by nucleic acid amplification techniques.

**8.** The method of claim 7, wherein the step (i) of determining the sequence of the C region of the genomic DNA of said OsHV-1 virus is done by PCR.

**9.** The method of any of claims 7 or 8, wherein the determining step (i) is done by PCR using the pair of primers:
- CR having the sequence GTG ATG GCT TTG GTC AAG GT (SEQ ID n°7), and
- CF having the sequence CCC CGG GGA AAA AGT ATA AA (SEQ ID n°8).

**10.** A pair of oligonucleotides useful for determining if a particular Ostreid herpesvirus 1 (OsHV-1) corresponds to the OsHV-1 µVar strain, wherein said pair of oligonucleotides has the sequences:
- GTG ATG GCT TTG GTC AAG GT (SEQ ID n°7), and
- CCC CGG GGA AAA AGT ATA AA (SEQ ID n°8).

**11.** A kit for determining if a particular Ostreid herpesvirus 1 (OsHV-1) corresponds to the OsHV-1 µVar strain, wherein said kit comprises the pair of oligonucleotides as defined in claim 10.
